# EUROPEAN PATENT APPLICATION

(11) **EP 4 631 965 A1**
(43) Date of publication of application: **15.10.2025**
(21) Application number: 22967979.0
(22) Date of filing: 16.12.2022
(51) Int. Cl.: C07K 7/06, A61K 9/127, A61P 19/02, A61L 27/22, A61K 38/00

(54) **PEPTIDE FOR CARTILAGE REGENERATION, AND USES THEREOF**

(30) Priority: 09.12.2022 KR 20220171942
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); YEON, Jeong Tae, Anyang-si, Gyeonggi-do 14119 (KR); CHO, Hyun Joo, Anyang-si, Gyeonggi-do 14119 (KR)
(74) Representative: Potter Clarkson
(86) International application number: PCT/KR2022/020555
(87) International publication number: WO 2024/122723

(57) **Abstract**

The present application relates to a peptide having a cartilage regeneration effect, and uses thereof, and provides a peptide including an amino acid sequence of SEQ ID NO: 1 or SEQ ID NO: 2, a composition for cartilage regeneration, including the peptide, and a pharmaceutical composition for preventing or treating a cartilage disease, including the composition for cartilage regeneration as an active ingredient.

## Description

### Technical Field

The present application relates to a peptide for cartilage regeneration, and uses thereof.

### Background Art

Due to the nature of cartilage tissue, when a large area is damaged, it is difficult to regenerate tissue through natural healing, and therefore, treatment has been performed through surgical procedures such as artificial joints, articular cartilage resurfacing, and microfracture. However, existing methods often leave scars due to incisions and cause regeneration into fibrocartilage with low durability, which has the problem of lower treatment effectiveness despite the difficult surgical method.

Accordingly, intra-articular injectable solutions or cartilage tissue repairing compositions using hydrogels, collagen, etc., which have a simple surgical procedure and quick therapeutic effects, have been developed (Korean Patent Publication No. 2013-0028012). However, although the above method may provide temporary pain relief, it is insufficient in inducing regeneration into cartilage tissue.

In addition, in the case of cell-based therapy, various treatment methods using autologous chondrocytes or stem cells, etc., have been developed as a method of inducing regeneration of cartilage tissue by transplanting cells cultured in vitro back into the defective area (Korean Patent Publication No. 2013-0072983). However, in the case of autologous chondrocyte treatment, there are limitations to treatment using only cells collected and cultured from the patient when the damaged area is large, and in the case of stem cell treatment, there are issues such as differences in cell number and differentiation potential depending on the area of collection, changes in cell phenotype due to cell dedifferentiation during in vitro culture, a low differentiation rate into chondrocytes after in vivo transplantation, and induction of vascular invasion along with cell apoptosis due to gene expression related to cell hypertrophy, which causes calcification of chondrocytes, etc.

Against this technical background, the development of effective factors that enable more effective treatment of cartilage diseases by promoting cartilage differentiation or cartilage formation of stem cells or chondrocytes is required, but is still insufficient.

### Disclosure of Invention

### Technical Problem

One aspect is to provide a peptide consisting of an amino acid sequence of SEQ ID NO: 1 or SEQ ID NO: 2.

Another aspect is to provide a composition for cartilage regeneration, including as an active ingredient a peptide consisting of an amino acid sequence of SEQ ID NO: 1 or SEQ ID NO: 2.

Another aspect is to provide a pharmaceutical composition for preventing or treating cartilage disease, including as an active ingredient a composition for cartilage regeneration.

Other objectives and advantages of the present application will become apparent from the following detailed description, together with the appended claims and drawings. Anything not set forth herein can be readily recognized and inferred by one skilled in the art or similar art and is hereby omitted.

### Solution to Problem

Each of the descriptions and embodiments disclosed in the present application may be applied to other descriptions and embodiments. In other words, all combinations of the various elements disclosed in the present application fall within the scope of the present application. Furthermore, the scope of the present application is not to be considered limited by the specific description set forth below.

One aspect provides a peptide consisting of an amino acid sequence of SEQ ID NO: 1 or SEQ ID NO: 2.

As used herein, the term "peptide" may refer to a linear molecule formed by combining amino acid residues to each other by peptide bonds. The peptides may be prepared according to chemical synthesis methods known in the art, in particular solid phase synthesis technique or liquid phase synthesis technique (US Registered Patent No. 5,516,891). As a result of diligent efforts to develop a peptide with biologically effective activity, the inventors of the present disclosure have identified a peptide consisting of an amino acid sequence of SEQ ID NO: 1 or SEQ ID NO: 2. Herein, the biologically effective activity may be any one or more selected from the following properties: (a) inducing production of glycosaminoglycan; (b) inducing production of COL2A1, Cartilage oligomeric matrix protein (COMP), COL11A or aggrecan; and (c) inducing production of regulatory factors SOX5, SOX6 or SOX9. Thus, the peptide may be utilized for uses for cartilage regeneration.

The peptide may have a protecting group bonded to the N- or C-terminus of the peptide to acquire chemical stability, enhanced pharmacological properties (half-life, absorption, titer, effect, etc.), altered specificity (for example, broad spectrum of biological activity), or reduced antigenicity. In an embodiment, an N-terminus of the peptide may be bonded with any one protecting group selected from the group consisting of an acetyl group, a fluoreonylmethoxycarbonyl group, a formyl group, a palmitoyl group, myristyl group, stearyl group, butoxycarbonyl group, allyloxycarbonyl group, and polyethylene glycol (PEG); and/or the C-terminus of the peptide may be bonded with any one protecting group selected from the group consisting of an amino group(-NH₂), a tertiary alkyl group, and an azide (-NHNH₂). Additionally, the peptide may optionally further include a targeting sequence, a tag, labeled residues, or an amino acid sequence prepared for the specific purpose of increasing the half-life or peptide stability.

The above peptide is artificially synthesized, or non-naturally occurring or engineered, and the "non-naturally occurring or engineered" refers to a state that is not in its natural state, but is created by artificial modification. Here, the artificial modification may include artificially synthesizing an amino acid sequence by mimicking a plurality of amino acid structures, or manipulating it to acquire chemical stability, enhanced pharmacological properties, altered specificity, or reduced antigenicity as described above.

As used herein, the term "stability" may refer to not only in vivo stability, which protects the peptide from attack by proteolytic enzymes in vivo, but also storage stability (for example, room temperature storage stability).

Another aspect provides a composition for cartilage regeneration, including as an active ingredient a peptide consisting of an amino acid sequence of SEQ ID NO: 1 or SEQ ID NO: 2.

In the above description of the peptide, the terms or elements referred to are the same as those already mentioned.

As used herein, the term "cartilage regeneration" may refer to restoring damaged cartilage tissue or inducing the creation of insufficient cartilage tissue to improve the cartilage tissue. As used herein, the term "ameliorate" may refer to any act that at least reduces a parameter associated with the alleviation or treatment of a condition, for example the severity of a symptom.

The cartilage includes, but is not limited to, hyaline cartilage, fibrocartilage or elastic cartilage. For example, the cartilage may be one or more selected from the group consisting of articular cartilage, ear cartilage, nasal cartilage, elbow cartilage, meniscus, knee cartilage, costal cartilage, ankle cartilage, tracheal cartilage, laryngeal cartilage and spinal cartilage.

Although prior functional peptides have effective biological activity, they have shown disadvantages such as not being able to effectively enter target tissue or cell due to the size of the peptide itself, or disappearing from the body in a short period of time due to their short half-life. In contrast, a cartilage regeneration composition according to an example includes, as an active ingredient, a peptide consisting of about 10 or less amino acids, and accordingly, the penetration rate, etc., of the active ingredient into the skin is very excellent, and may provide an effective cartilage regeneration effect, for example, when administered topically.

According to an embodiment, the peptide may significantly increase the expression of glycosaminoglycan, COL2A1, COMP, COL11A, aggrecan, regulatory factor SOX5, SOX6 or SOX9, which are substances related to cartilage, and thus, the peptide may be utilized as an active ingredient of a composition for cartilage regeneration (Orthop Res Rev. 2010 September 1; 2010(2): 85-94. doi:10.2147/ORR.S7194, JOSPT Volume 28 Number 4 October 1998).

Another aspect provides a pharmaceutical composition for preventing or treating cartilage disease, including, as an active ingredient, a peptide consisting of an amino acid sequence of SEQ ID NO: 1 or SEQ ID NO: 2.

In the above description of the peptide or composition, the terms or elements referred to are the same as those already mentioned.

As used herein, the term "prevention" refers to any act of inhibiting or delaying the onset of a disease by administering the composition.

As used herein, the term "treatment" refers to any form of treatment that provides an effect to a subject suffering from a disease or at risk of developing a disease, including improving the condition of the subject (for example, one or more symptoms), delaying the progression of the disease, delaying the onset of symptoms, or slowing the progression of symptoms, etc. Therefore, the above "treatment" and "prevention" are not intended to refer to cure or complete elimination of symptoms.

The above "individual" refers to a person in need of treatment of a disease, and more specifically, a mammal, such as a human or non-human primate, mouse, dog, cat, horse, and bovine, etc.

As used herein, the term "cartilage disease" refers to any disease related to cartilage that requires cartilage differentiation or regeneration. The cartilage disease may be one or more selected from the group consisting of cartilage damage, cartilage defect, degenerative disc disease, intervertebral disc herniation, degenerative arthritis, fracture, damage to muscle tissue, nonunion of fracture or joint damage due to trauma, osteomalacia and chondromalacia.

The cartilage diseases may occur in the temporomandibular joint, shoulder joint, elbow joint, wrist joint, finger joint, spinal joint, hip joint, knee joint, ankle joint, or toe joint.

The pharmaceutical composition may include, a pharmaceutically effective amount of the peptide; and/or a pharmaceutically acceptable carrier, but is not limited thereto.

As used herein, the term "pharmaceutically effective amount" may refer to an amount sufficient to achieve the cartilage regeneration efficacy of the pharmaceutical composition.

The weight ratio between the peptide and a pharmaceutically acceptable carrier may be, for example, 500:1 to 1:500, for instance, the weight ratio may be 450:1 to 1:450, 400:1 to 1:400, 350:1 to 1:350, 300:1 to 1:300, 250:1 to 1:250, 200: 1 to 1:200, 150:1 to 1:150, 100:1 to 1:100, 80:1 to 1:80, 60:1 to 1:60, 40:1 to 1:40, 20:1 to 1:20, 10:1 to 1:10, 8:1 to 1:8, 6:1 to 1:6, 4:1 to 1:4, or 2:1 to 1:2, but is not limited thereto.

The pharmaceutically acceptable carrier is commonly used in formulations and include, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, etc., but is not limited thereto. Suitable pharmaceutically acceptable carriers and formulations are described in detail in Remington's Pharmaceutical Sciences (19th ed., 1995).

In addition to the above ingredients, the pharmaceutical composition may additionally include, lubricants, humectant, sweetener, flavoring agent, emulsifier, suspending agent, preservative, etc., but is not limited thereto.

The pharmaceutical composition may be administered orally or parenterally, preferably parenterally, and if parenterally administered, may be administered by, but not limited to, intramuscular injection, intravenous injection, subcutaneous injection, intraperitoneal injection, topical administration, transdermal administration, etc.

The dosage of the pharmaceutical composition may be, but is not limited to, 0.0001 to 1000 ug (micrograms), 0.001 to 1000 ug, 0.01 to 1000 ug, 0.1 to 1000 ug, or 1.0 to 1000 ug per day, and may be varied by factors such as method of formulation, mode of administration, patient age, weight, sex, medical condition, food, time of administration, route of administration, rate of excretion, and response sensitivity.

The pharmaceutical composition may be formulated, with a pharmaceutically acceptable carrier and/or excipient, according to a method that could be readily practiced by a person skilled in the art to which the present disclosure belongs, and the composition may be prepared into a unit dosage form or may be contained in a multi-dose container.

The formulation may be in the form of a solution, suspension or emulsion in an oil or aqueous medium, or in the form of an ointment, cream, gel, transdermal absorbent, cataplasma, patch, paste, extract, powder, granule, tablet or capsule, and may additionally include a dispersant and/or a stabilizer.

The peptide may be incorporated into a nanosome or a nanoparticle to further improve skin penetration or stability issues. For example, the nanosome may be prepared by a microfluidizer using lecithin as a raw material, and may be incorporated in lecithin particles. Any method of preparing the nanosome may be used as long as it is known. The size of the nanosome particle is preferably 30 to 200 nm. If the size of the nanosome particle is less than 30 nm, skin penetration may progress very quickly, resulting in skin side effects, and if the size is greater than 200 nm, skin penetration may not be easy, making it difficult to achieve the effect of using the nanosome structure.

Another aspect provides a method of preventing or treating a cartilage disease, including administering to a subject a pharmaceutical composition including, as an active ingredient, a therapeutically effective amount of a peptide consisting of an amino acid sequence of SEQ ID NO: 1 or SEQ ID NO: 2.

In the above description of the peptide, composition, etc., the terms or elements referred to are the same as those already mentioned.

As used herein, the terms "applying," "administering," and "applying" are used interchangeably and may refer to causing at least partial localization of a composition according to an example to a desired site, or to placing a composition according to an example into an individual by route of administration.

Another aspect provides a cosmetic composition, including as an active ingredient a peptide consisting of an amino acid sequence of SEQ ID NO: 1 or SEQ ID NO: 2.

In the above description of the peptide, composition, etc., the terms or elements referred to are the same as those already mentioned.

The cosmetic composition may include, a cosmetically effective amount of the peptide; and/or a cosmetically acceptable carrier, but is not limited thereto.

Another aspect provides a method of regenerating cartilage, including administering to a subject a composition including, as an active ingredient, a peptide consisting of an amino acid sequence of SEQ ID NO: 1 or SEQ ID NO: 2.

In the above description of the peptide, composition, etc., the terms or elements referred to are the same as those already mentioned.

### Advantageous Effects of Invention

According to a peptide according to an aspect, the peptide exhibits an excellent cartilage regeneration effect by significantly increasing various cartilage components such as glycosaminoglycan, collagen, COMP, and aggrecan, etc.

According to a peptide according to an aspect, the peptide may be applied to prevent or treat cartilage disorders and promote cartilage regeneration by significantly increasing various cartilage components such as glycosaminoglycan, collagen, COMP, and aggrecan, etc.

### Brief Description of Drawings

FIG. 1 shows the results of confirming CCK-8 activity after treating C28/I2 cells with Peptide-1.
FIG. 2 shows the results of confirming CCK-8 activity after treating C28/I2 cells with Peptide-2.
FIG. 3 shows the results of confirming increases in the production of glycosaminoglycan after treating C28/I2 cells with Peptide-1.
FIG. 4 shows the results of confirming increases in the production of glycosaminoglycan after treating C28/I2 cells with Peptide-2.
FIG. 5 shows the results of confirming increases in mRNA expression of ECM components after treating C28/I2 cells with Peptide-1.
FIG. 6 shows the results of confirming increases in mRNA expression of ECM components after treating C28/I2 cells with Peptide-2.
FIG. 7 shows the results of confirming increases in mRNA expression of SOX9, an ECM regulatory factor, after treating C28/I2 cells with Peptide-1.
FIG. 8 shows the results of confirming increases in mRNA expression of SOX9, an ECM regulatory factor, after treating C28/I2 cells with Peptide-2.
FIG. 9 shows the results of confirming increased expression of SOX9 protein, an ECM regulatory factor, after treating C28/I2 cells with Peptide-1.
FIG. 10 shows the results of confirming increased expression of ECM regulatory factors SOX5, SOX6, and SOX9 proteins after treating C28/I2 cells with Peptide-2.

### Mode for the Invention

Hereinafter, the present disclosure will be described in more detail with reference to examples. However, these examples are intended to illustrate the present disclosure by way of example and the scope of the present disclosure is not limited to these examples.

### Example 1. Synthesis of Peptide

Using an automatic peptide synthesizer (Milligen 9050, Millipore, USA), peptides (Peptide-1 or Peptide-2) having amino acid sequences of SEQ ID NO: 1 or 2 as shown in Table 1 below were synthesized, and these synthesized peptides were purified using C18 reverse-phase high-performance liquid chromatography (HPLC) (Waters Associates, USA). The column used was an ACQUITY UPLC BEH300 C18 (2.1 mm X 100 mm, 1.7 µm, Waters Co, USA).

**[Table 1]**

| **Name** | **Amino acid sequence (N terminus - > C terminus)** | **SEQ ID NO:** |
|---|---|---|
| Peptide-1 | PYYRI | 1 |
| Peptide-2 | KFLIK | 2 |

### Example 2. Confirmation of Cytotoxicity

The cytotoxicity of Peptide-1 or Peptide-2 upon addition to human-derived chondrocytes (C28/I2, human chondrocyte cell line) was analyzed using a CCK-8 assay.

Specifically, human-derived chondrocytes were seeded in a 96-well plate at a density of 3 × 10³ cells/well and cultured in DMEM medium (cat. 11995-065, Gibco) for 24 hours. Then, after replacing with a new medium, Peptide-1 or Peptide-2 was treated at different concentrations, respectively. Then, after 3 days, CCK-8 (Dojindo, CCK-8 kit) solution was added at 1/10 volume of the culture medium and incubated for 2 hours. The culture medium was sampled and CCK-8 activity was confirmed at a wavelength of 450 nm using a microplate reader. Meanwhile, as a negative control group, an experimental group to which the peptide was not added was used.

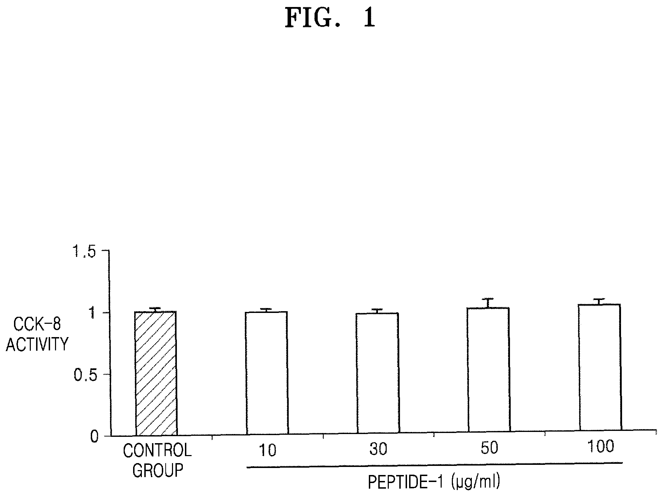

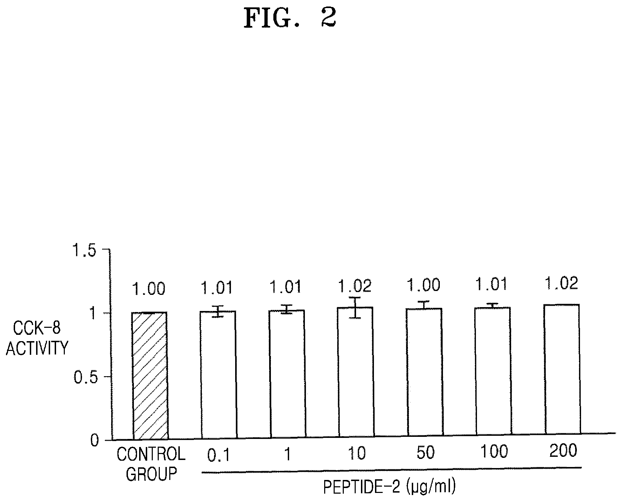

As a result, as shown in FIG. 1 and FIG. 2, it was found that each of Peptide-1 and Peptide-2 did not exhibit toxicity in human-derived chondrocytes.

### Example 3. Confirmation of Glycosaminoglycan Production Effect

By confirming the effect of increasing the production of glycosaminoglycan by adding Peptide-1 or Peptide-2, respectively, to human-derived chondrocytes, the effects of the peptides on inducing cartilage production and promoting extracellular matrix (ECM) production were confirmed.

Specifically, human-derived chondrocytes were seeded in a 96-well plate at a density of 3 × 10³ cells/well and cultured in DMEM medium (cat. 11995-065, Gibco) for 24 hours. Then, after replacing with a new medium, Peptide-1 or Peptide-2 was treated at different concentrations, respectively. Subsequently, the medium was replaced every 3 days and Peptide-1 or Peptide-2 was treated at different concentrations, respectively. After 7 days, the medium were suctioned and 60 µL of 3.7 % formalin was added to the 96-well plate to be stained and fixed for 1 minute. After suctioning 3.7 % formalin, 70 µL of Alcian blue staining solution (3 % acetic acid 50 mL + 1 % Alcian blue 8GX 0.5 g, pH 2.5) was dispensed. After incubation at 37 °C for 24 hours, the staining solution was suctioned, washed with distilled water, dried, and observed under a microscope. Meanwhile, as a positive control group, a group to which TGFβ1 (20 ng/ml) was added was used. Meanwhile, as a negative control group, an experimental group to which the peptide was not added was used, and as a positive control group, an experimental group to which TGF-β was added was used.

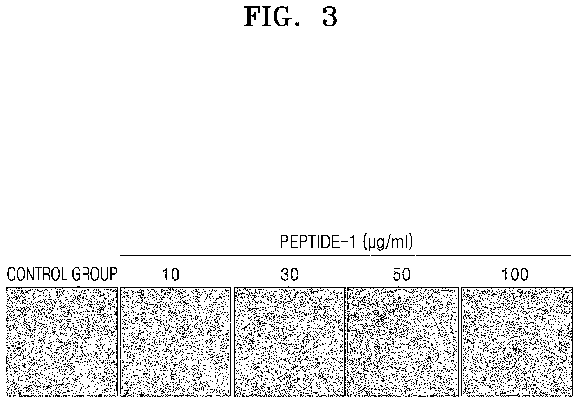

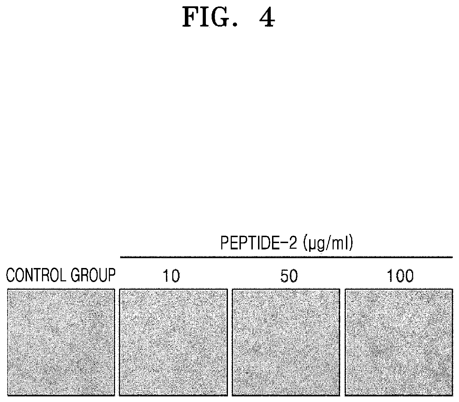

As a result, as shown in FIG. 3 and FIG. 4, it was found that each of Peptide-1 and Peptide-2 promoted the production of glycosaminoglycan.

### Example 4. Confirmation of Increased mRNA Expression of ECM Components

The aim was to determine whether treatment with Peptide-1 or Peptide-2, respectively, increased the mRNA expression of ECM components in human-derived chondrocytes.

Specifically, human-derived chondrocytes were seeded in a 6-well plate at a density of 8.9 × 10⁴ cells/well and cultured in DMEM medium (cat. 11995-065, Gibco) for 24 hours. Then, after replacing with a new medium, Peptide-1 or Peptide-2 was treated at different concentrations, respectively. Subsequently, the medium was replaced every 3 days and Peptide-1 or Peptide-2 was treated at different concentrations, respectively. After 1, 3, and 7 days, the medium was suctioned, cells were harvested, and RNA was isolated. After synthesizing cDNA using a cDNA synthesis kit & PCR pre-mix (Intron, Korea), PCR was performed using the hCOL2A1, COMP, hCOL11A, ACAN, and GAPDH primers shown in Table 2 below. Meanwhile, as a negative control group, an experimental group to which the peptide was not added was used, and as a positive control group, an experimental group to which TGF-β was added was used. In Table 2 below, hCOL2A1 encodes collagen type II alpha 1, hCOMP encodes cartilage oligomeric matrix protein, hCOL11A encodes the alpha chain of collagen type XI, hACAN encodes aggrecan, and hGAPDH encodes glyceraldehyde-3-phosphate dehydrogenase.

**[Table 2]**

| **Primer Name** | | **Sequence (5'->3')** | **SEQ ID NO:** |
|---|---|---|---|
| hCOL2A1 | Forward | CTATCTGGACGAAGCAGCTGGCA | 3 |
| | Reverse | ATGGGTGCAATGTCAATGATGG | 4 |
| hCOMP | Forward | AACTCAGGGCAGGAGGATGT | 5 |
| | Reverse | TGTCCTTTTGGTCGTCGTTC | 6 |
| hCOL11A | Forward | CCAGCCCGAACTTGTAAAGA | 7 |
| | Reverse | TGGACGCACAACCATCATAC | 8 |
| hACAN | Forward | GCAGGCTATGAGCAGTGTGA | 9 |
| | Reverse | GCATACCTCGGAAGCAGAAG | 10 |
| hGAPDH | Forward | ACCACAGTCCATGCCATCAC | 11 |
| | Reverse | TCCACCACCCTGTTGCTGTA | 12 |

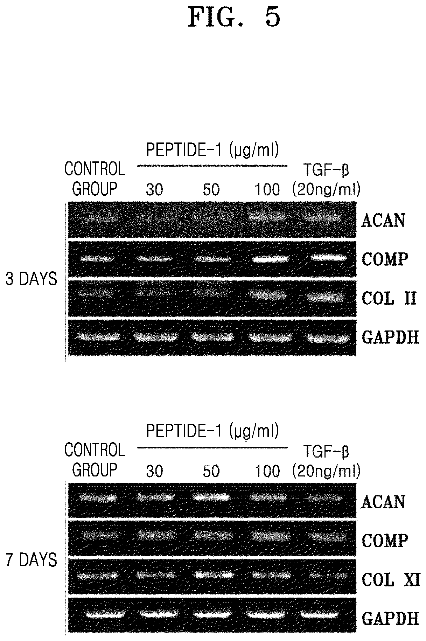

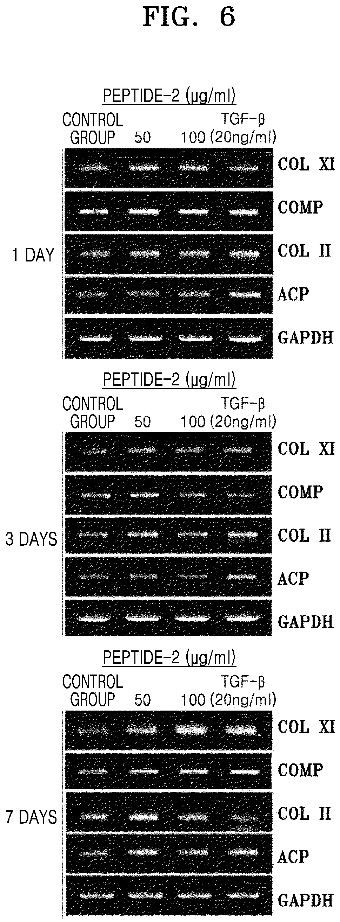

As a result, as shown in FIG. 5 and FIG. 6, it was found that each of Peptide-1 and Peptide-2 increased the mRNA production of ECMs COL2A1, COMP, COL11A, and ACAN.

### Example 5. Confirmation of mRNA Expression Induction Effect of ECM Regulatory Factor SOX9

The aim was to determine whether treatment with Peptide-1 or Peptide-2, increased the mRNA expression of ECM regulatory factor SOX9 in human-derived chondrocytes.

Specifically, human-derived chondrocytes were seeded in a 6-well plate at a density of 8.9 × 10⁴ cells/well and cultured in DMEM medium (cat. 11995-065, Gibco) for 24 hours. Then, after replacing with a new medium, Peptide-1 or Peptide-2 was treated at different concentrations, respectively. Subsequently, the medium was replaced every 3 days and Peptide-1 or Peptide-2 was treated at different concentrations, respectively. After 1, 3, and 7 days, the medium was suctioned, cells were harvested, and RNA was isolated. After synthesizing cDNA using a cDNA synthesis kit & PCR pre-mix (Intron, Korea), PCR was performed using the primers shown in Table 3 below. Meanwhile, as a negative control group, an experimental group to which the peptide was not added was used, and as a positive control group, an experimental group to which TGF-β was added was used. In Table 3 below, SOX9 refers to (sex determining region Y)-box 9.

**[Table 3]**

| **Primer Name** | | **Sequence (5'->3')** | **SEQ ID NO:** |
|---|---|---|---|
| hSOX9 | Forward | GCGGAGGAAGTCGGTGAAGA | 13 |
| | Reverse | CCCTCTCGCTTCAGGTCAGC | 14 |
| hGAPDH | Forward | ACCACAGTCCATGCCATCAC | 11 |
| | Reverse | TCCACCACCCTGTTGCTGTA | 12 |

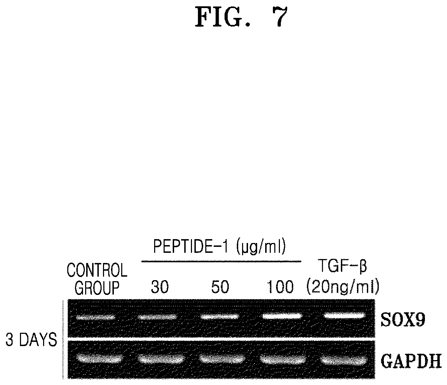

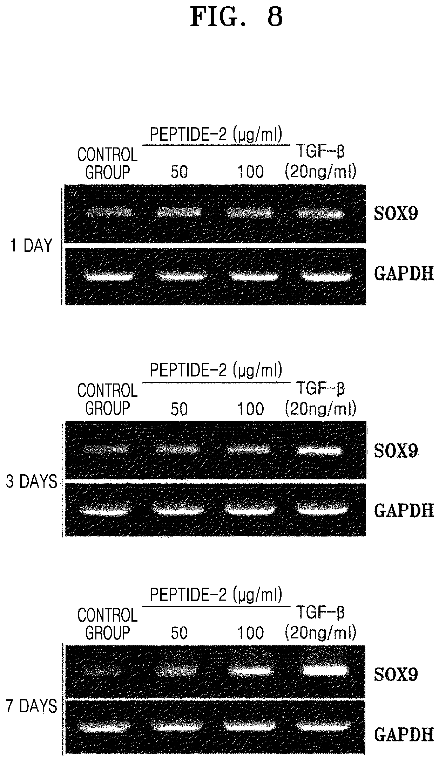

As a result, as shown in FIG. 7 and FIG. 8, it was found that each of Peptide-1 and Peptide-2 induced the production of SOX9, an ECM regulatory factor.

### Example 6. Confirmation of Expression Induction Effect of ECM Regulatory Factors SOX5, SOX6, and SOX9

The aim was to determine whether treatment with Peptide-1 or Peptide-2, respectively, increased the expression of ECM regulatory factors SOX5, SOX6, and SOX9 in human-derived chondrocytes.

Specifically, human-derived chondrocytes were seeded in a 6-well plate at a density of 8.9 × 10⁴ cells/well and cultured in DMEM medium (cat. 11995-065, Gibco) for 24 hours. Then, after replacing with a new medium, Peptide-1 or Peptide-2 was treated at different concentrations, respectively. Subsequently, the medium was replaced every 3 days and Peptide-1 or Peptide-2 was treated at different concentrations, respectively. After 1, 3, and 7 days, the medium was suctioned, cells were harvested, lysates were prepared, and Western blot was performed. As antibodies for detection, sc-293215 (Santa Cruz, USA) for SOX5, sc-393314 (Santa Cruz, USA) for SOX6, and 82630S (Cell Signaling, USA) for SOX9 were used. Meanwhile, as a negative control group, an experimental group to which the peptide was not added was used, and as a positive control group, an experimental group to which TGF-β was added was used.

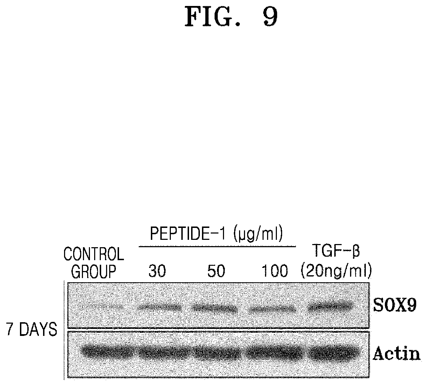

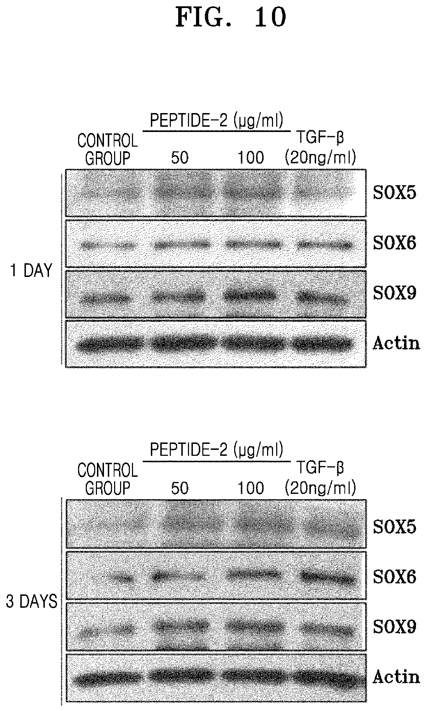

As a result, as shown in FIG. 9 and FIG. 10, it was found that each of Peptide-1 and Peptide-2 increased the expression of ECM regulatory factors SOX5, SOX6, and SOX9.

In summary of the above experimental results, it was found that each of Peptide-1 and Peptide-2 according to an embodiment has an effect of inducing cartilage regeneration.

### Formulation Example 1. Preparation of Peptide Nanosomes

50 mg of the peptide of Example 1 above was dissolved in 500 ml of distilled water by sufficient stirring. The composite solution was mixed with 5 g of lecithin, 0.3 ml of sodium oleate, 50 ml of ethanol, and a small amount of oil, and the total amount was adjusted to 1 L with distilled water, and the mixture was then emulsified using a mat high pressure to produce peptide nanosomes with a size of about 100 nm.

### Formulation Example 2. Pharmaceutical Preparation

### 2-1. Preparation of Powder

A powder is prepared by mixing the following ingredients and filling them into airtight pouches.
Peptide of the present disclosure 20 mg
Lactose 100 mg
Talc 10 mg

### 2-2. Preparation of Tablet

A tablet is prepared by mixing the following ingredients and compressing them according to a usual method of tablet preparation.
Peptide of the present disclosure 10 mg
Corn starch 100 mg
Lactose 100 mg
Magnesium stearate 2 mg

### 2-3. Preparation of Capsule

A capsule is prepared by mixing the following ingredients according to a usual method of capsule preparation and filling the mixture into gelatin capsules.
Peptide of the present disclosure 10 mg
Crystalline cellulose 3 mg
Lactose 14.8 mg
Magnesium stearate 0.2 mg

### 2-4. Preparation of Injection

An injection is prepared according to a usual method of injection preparation such that the following ingredients are contained per 2 ml ampoule.
Peptide of the present disclosure 10 mg
Mannitol 180 mg
Sterile distilled water for injection 2974 mg
Na₂HPO₄·2H2O 26 mg

### 2-5. Preparation of Liquid Formulation

According to the usual method of preparing a liquid, each ingredient is added to purified water and dissolved, the following ingredients are mixed, purified water is added to adjust the total to 100 ml, and the liquid is then filled into a brown bottle and sterilized to prepare the liquid.
Peptide of the present disclosure 10 mg
Isomerized sugar 10 g
Mannitol 5 g
Appropriate amount of purified water

The foregoing description of the present disclosure is for illustrative purposes only, and one that has ordinary skill in the art to which the present disclosure belongs will understand that it may be readily adapted to other specific forms without altering the technical ideas or essential features of the present disclosure. Therefore, the examples described above should be understood in all respects as illustrative and not restrictive.

## Claims

1. A peptide consisting of an amino acid sequence of SEQ ID NO: 1 or SEQ ID NO: 2.

2. The peptide of claim 1, wherein an N-terminus of the peptide is bonded to any one protecting group selected from the group consisting of an acetyl group, fluoreonylmethoxycarbonyl group, formyl group, palmitoyl group, myristyl group, stearyl group, butoxycarbonyl group, allyloxycarbonyl group, and polyethylene glycol (PEG).

3. The peptide of claim 1, wherein a C-terminus of the peptide is bonded to any one protecting group selected from the group consisting of an amino group (-NH₂), tertiary alkyl group, and azide (-NHNH₂).

4. The peptide of claim 1, wherein the peptide exhibits any one or more selected from the following characteristics:
(a) inducing production of glycosaminoglycan;
(b) inducing production of COL2A1, COMP, COL11A, or aggrecan; and
(c) inducing production of SOX5, SOX6, or SOX9.

5. A composition for cartilage regeneration, comprising, as an active ingredient, a peptide according to any one of claims 1 to 4.

6. A pharmaceutical composition for preventing or treating a cartilage disease, comprising, as an active ingredient, a peptide according to any one of claims 1 to 4.

7. The pharmaceutical composition of claim 6, further comprising a pharmaceutically acceptable carrier.

8. The pharmaceutical composition of claim 6, wherein the peptide is formulated in nanosome form.

9. The pharmaceutical composition of claim 6, wherein the cartilage disease is one or more selected from the group consisting of cartilage damage, cartilage defect, degenerative disc disease, intervertebral disc herniation, degenerative arthritis, fracture, damage to muscle tissue, nonunion of fracture or joint damage due to trauma, osteomalacia, and chondromalacia.
